# EUROPEAN PATENT APPLICATION

(11) **EP 3 572 406 A1**
(43) Date of publication of application: **27.11.2019**
(21) Application number: 18201039.7
(22) Date of filing: 17.10.2018
(51) Int. Cl.: C07D 239/54, A61P 5/02, A61K 31/513

(54) **ACID ADDITION SALT OF ELAGOLIX AND RELATED COMPOUNDS**

(71) Applicant: SANDOZ AG, 4056 Basel (CH)
(72) Inventor: SCHREINER, Erwin, 6250 Kundl (AT); FELZMANN, Wolfgang, 6250 Kundl (AT); NERDINGER, Sven, 6250 Kundl (AT); MARTIN, Nolwenn, 6250 Kundl (AT)
(74) Representative: Prüfer & Partner mbB Patentanwälte · Rechtsanwälte

(57) **Abstract**

The current invention discloses preparation of acid addition salts of 4-[2-[5-(2-Fluoro-3-methoxyphenyl)-3-[2-fluoro-6-(trifluoromethyl)benzyl]-4-methyl-2,6-dioxo-1,2,3,6-tetrahydro-1-pyrimidinyl]-1(R)-phenylethylamino]butyric acid by treatment of an acid-sensitive ester of , 4-[2-[5-(2-Fluoro-3-methoxyphenyl)-3-[2-fluoro-6-(trifluoromethyl)benzyl]-4-methyl-2,6-dioxo-1,2,3,6-tetrahydro-1-pyrimidinyl]-1 (R)-phenylethylamino]butyric acid with a sufficiently strong acid and subsequent isolation by filtration. The process of the invention prevents formation of (R)-5-(2-fluoro-3-methoxyphenyl)-1-(2-fluoro-6-(trifluoromethyl)benzyl)-6-methyl-3-(2-(2-oxopyrrolidin-1-yl)-2-phenylethyl)pyrimidine-2,4(1H,3H)-dione (Va).

## Description

### FIELD OF THE INVENTION

The present invention relates to processes for the preparation of acid addition salts of the gonadotropin-releasing hormone receptor antagonist elagolix and related compounds and pharmaceutical compositions comprising same. It also refers to said pharmaceutical compositions for use in a medical treatment.

### BACKGROUND OF THE INVENTION

Elagolix is an orally available gonadotropin-releasing hormone (GnRH) receptor antagonist currently under investigation in clinical phase III trials for the treatment of endometriosis and uterine fibroids. The chemical name of elagolix is 4-[[(1 R)-2-[5-(2-fluoro-3-methoxyphenyl)-3-[[2-fluoro-6-(trifluoromethyl)phenyl]methyl]-3,6-dihydro-4-methyl-2,6-dioxo-1(2H)-pyrimidinyl]-1-phenylethyl]amino]butanoic acid. Elagolix is an uracil derivative which can be represented by the chemical structure according to Formula (Ia)

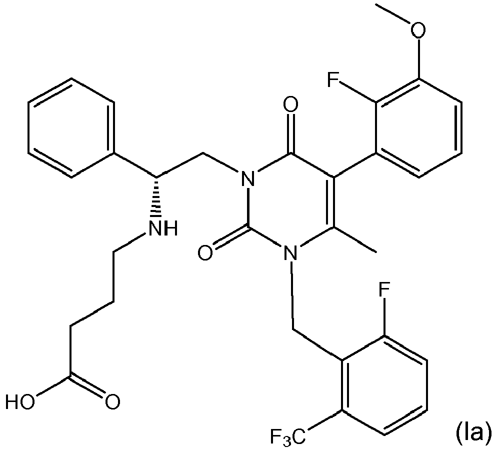

Related compounds of elagolix and their preparation are disclosed in WO 2005/007165 A1 and WO 2009/062087 A1, respectively. Related compounds of elagolix have the general Formula (1) or Formula (I): 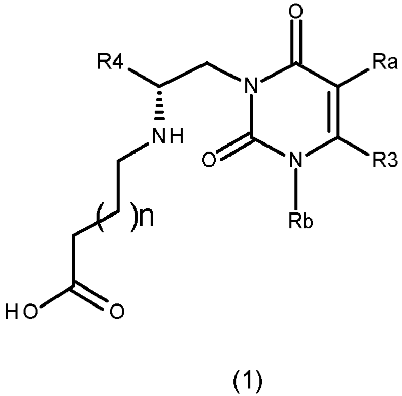 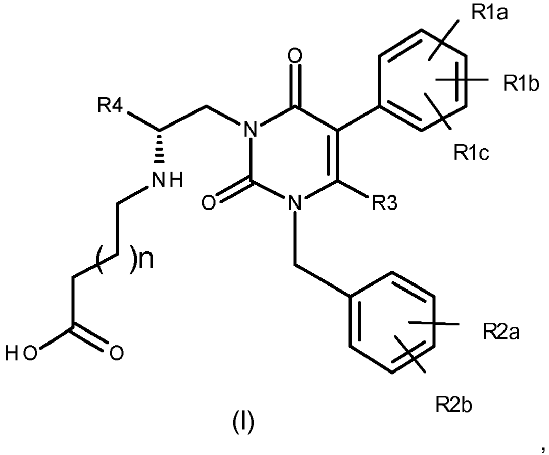 wherein the substituents are as defined for the acid addition salt of Formula (2) and Formula (II) herein, respectively.

Elagolix has received regulatory approval recently in US and is now marketed as tablets comprising its amorphous sodium salt.

WO 2005/007165 A1 discloses pyrimidine-2,4-dione derivatives as gonadotropin-releasing hormone receptor antagonists. The publication mentions on page 12 that said compounds may generally be used as the free acid or free base or alternatively in form of acid or base addition salts followed by a long list of potentially suitable acids and bases. The compound elagolix is disclosed as one example of pyrimidine-2,4-dione derivatives. In Example 1 H of said application elagolix free acid was formed as an intermediate during production of the elagolix sodium salt and was described as a white gel after extraction with ethyl acetate and evaporation of the solvent. The gel was passed through a DOWEX MSC-1 macroporous strong cation- exchange column to convert the acid to the sodium salt. Finally, lyophilization gave the sodium salt of elagolix as a white solid.

According to Chen C. et al. "Discovery of Sodium R-(+)-4-{2-[5-(2-Fluoro-3-methoxyphenyl)-3-(2-fluoro-6-[trifluoromethyl]benzyl)-4-methyl-2,6-dioxo-3,6-dihydro-2H-pyrimidin-1-yl]-1-phenylethylamino}butyrate (Elagolix), a Potent and Orally Available Nonpeptide Antagonist of the Human Gonadotropin-Releasing Hormone Receptor" J. Med. Chem., 2008, 51 (23), 7478-7485 elagolix free acid was either obtained as white foam or as gel after extraction with ethyl acetate and subsequent evaporation of the solvent. As already previously described in WO 2005/007165 A1 the gel was passed through a DOWEX MSC-1 macroporous strong cation-exchange column to convert it to the sodium salt, which was obtained as white solid after lyophilization.

WO 2009/062087 A1 describes processes for the preparation of uracil derivatives, a class of gonadotropin-releasing hormone receptor antagonists including elagolix. Elagolix free acid (Example 4, final product of step 4B) and its sodium salt (Example 5, final product of step 5B and alternate step 5B) were both obtained as off-white solids. On page 6, the application describes the compounds of the application as amorphous solids and goes on to suggest that the compounds of the application are formulated as amorphous co-solutions, for example by spray-drying with excipients such as PVP (polyvinyl pyrrolidone, Kollidons) or HPMC (hydroxypropylmethylcellulose). Amorphous elagolix sodium is described as preferred for that purpose and in Example 9 a solid amorphous mixture was prepared from elagolix sodium and polymers such as HPMC and Kollidon, wherein an excess of polymer at a weight ratio of 1:3 has been used.

It is noteworthy that elagolix (or related compounds) or its pharmaceutically acceptable salts have not been obtained as crystalline materials so far during pharmaceutical development despite what must have been considerable efforts. Even worse, elagolix sodium is highly hygroscopic and was found to deliquesce upon moisture contact. Such properties requiring special care and precautionary measures, such as controlled atmosphere during pharmaceutical processing, which renders manufacturing cumbersome and expensive.

In the unpublished application PCT/EP2018/059218 it was found that salts of elagolix with strong acids, in particular wherein the stoichiometry of elagolix : anion is about 1:1, can be obtained which do not deliquesce and /or show unexpected low hygroscopicity, in particular when compared to the widely used salts of elagolix with a base, like the elagolix sodium salt. During preparation of these acid addition salts of elagolix it was also surprisingly found that elagolix-like compounds have a strong tendency towards auto-cyclization, thus generating lactames corresponding to compound Va as an impurity in detectable amounts.

### SUMMARY OF THE INVENTION

Elagolix or related compounds prepared by the processes disclosed in the prior art are obtained as sodium salts, formed by alkaline cleavage of an ethyl ester. When these were transformed into acid additions salt of e.g., elagolix (Formula IIa), this transformation involved formation of elagolix free acid.

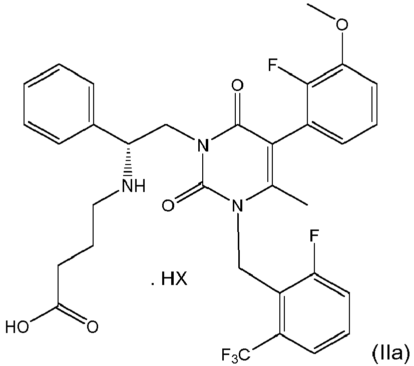

It came as a surprise that elagolix in free undissociated form, however, transforms auto-catalytically into the lactame of Formula (Va), (R)-5-(2-fluoro-3-methoxyphenyl)-1-(2-fluoro-6-(trifluoromethyl)benzyl)-6-methyl-3-(2-(2-oxopyrrolidin-1-yl)-2-phenylethyl)pyrimidine-2,4(1H,3H)-dione, which therefore has to be considered as an intrinsic impurity formed in the course of this process.

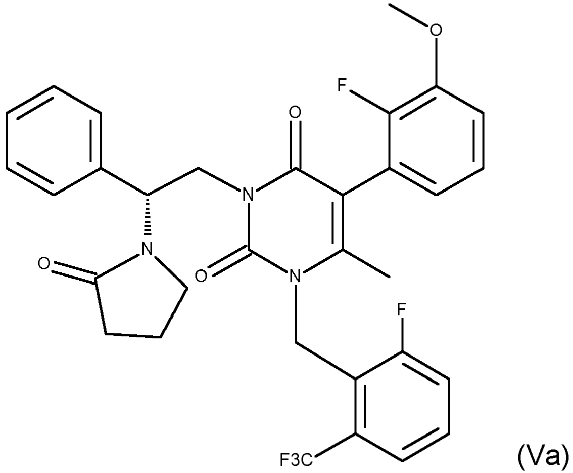

The corresponding impurities of Formula (5)/(V) can be expected to occur when preparing an acid addition salt of a compound of Formula (1) / I by a process that proceeds via the related free undissociated form of Formula (1)/(I), as well.

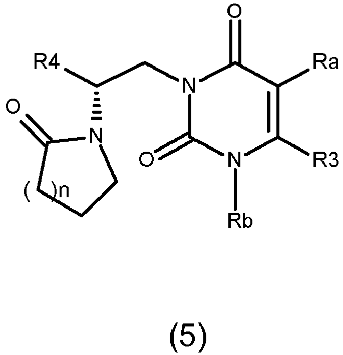

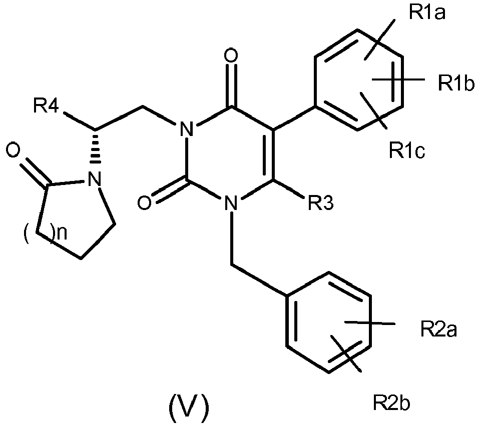

Without wishing to be bound by any theory, it is possible that the particular spatial conformation of the secondary amino group of elagolix, being in proximity to the uracil ring, might be responsible for this extraordinary sensitivity towards lactame formation.

In order to prevent the formation of these impurities, we aimed at development of a process for the preparation of acid addition salts of elagolix and related compounds - compounds of formula (1) / I - without involving the free undissociated form of the acid. This was realized by treatment of an acid-sensitive ester of elagolix (Formula IIIa) with a sufficiently strong acid (pKa <4, preferably <3, particularly preferred <2), which first, by a very fast protonation reaction, masks the secondary amine by forming the intermediate of Formula (IIIb) and thereby prevents it to engage in lactame formation. Then, at a much lower rate the sufficiently strong acid cleaves the acid-sensitive ester by forming the acid addition salt of elagolix.

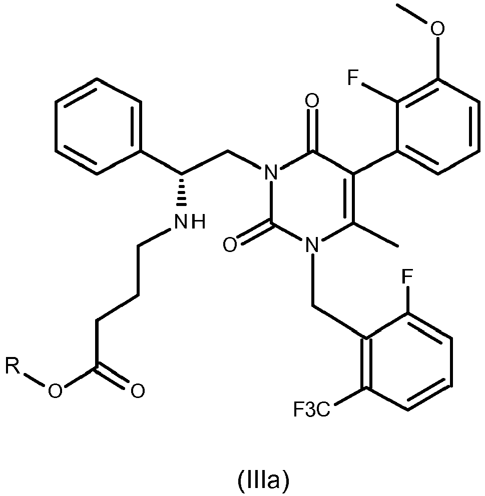

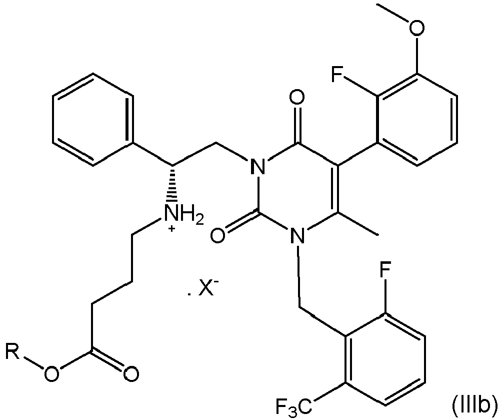

### General terms:

Listed below are definitions of various terms used to describe the present invention. These definitions, either by replacing one, more than one or all general expressions or symbols used in the present disclosure and thus yielding preferred embodiments of the invention, preferably apply to the terms as they are used throughout the specification unless they are otherwise limited in specific instances either individually or as part of a larger group.

The term "alkyl" means a straight chain or branched, noncyclic or cyclic hydrocarbon. Representative straight chain alkyls include methyl, ethyl, n-propyl, n-butyl, and the like; branched alkyls include isopropyl, sec-butyl, isobutyl, tert-butyl, and the like; while cyclic alkyls include cyclopropyl and the like.

The term "alkoxy" means "-O-alkyl".

The term "halogen" means fluoride, chloride, bromide or iodide, preferably fluoride, chloride or bromide.

The term "alkanediyl" means a divalent alkyl from which two hydrogen atoms are taken from the same carbon atom or from different carbon atoms, for example -CH₂-, -CH₂CH₂-, -CH₂CH₂CH₂-, -CH(CH₃)CH₂CH₂-, or -CH₂C(CH₃)₂CH₂-.

Protecting groups may be present and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products. The specialist knows, or can easily establish, which protecting groups are suitable with the reactions mentioned hereinabove and hereinafter. Preferably, if two or more protecting groups are present in one intermediate mentioned herein, they are chosen so that, if one of the groups needs to be removed, this can be done selectively, e.g., using two or more different protecting groups that are cleavable under different conditions, e.g. one class by mild hydrolysis, the other by hydrolysis under harder conditions, one class by hydrolysis in the presence of an acid, the other by hydrolysis in the presence of a base, or one class by reductive cleavage (e.g., by catalytic hydrogenation), the other by hydrolysis, or the like.

Nitrogen protecting groups, as defined above, can be introduced/removed according to standard methods of organic chemistry known in the art, in particular reference is made to conventional nitrogen protecting group methods described in J. F. W. McOmie, "Protective Groups in Organic Chemistry", Plenum Press, London and New York 1973, T. W. Greene and P. G. M. Wuts, "Greene's Protective Groups in Organic Synthesis", Fourth Edition, Wiley, New York 2007; and in Richard C. Larock, "Comprehensive Organic Transformations: A Guide to Functional Group Preparations", Second Edition, Wiley-VCH Verlag GmbH, 2000.
Hydroxyl protecting groups can be introduced/removed according to known methods; standard conditions for such methods are described, for example in reference books above-mentioned for nitrogen protecting groups.

The term "saponification reagent" is to be understood as a base which is able to hydrolyze an ester to form an alcohol and the salt of a carboxylic acid, e.g., an alkali metal hydroxide such as KOH or NaOH.

The term "group which can be saponified" is to be understood as an ester group -CO2R wherein R is alkyl, aryl or arylalkyl, which can be hydrolized, for example under basic conditions (e.g. alkalimetal base such as. NaOH, LiOH or KOH) or under acidic conditions (e.g., by the use of mineral acids, such as HCl, H2SO4, HBr, H3PO4) to provide a carboxylic acid. As an extension, the term "group which can be saponified" can also include an ester group -CO2R wherein R is aryl or arylalkyl, which can be reacted by use of a hydrogenation catalyst (e.g., Pd/C, Pt/C, Rh/C, Pd/Al2O3, PtO2), in the presence of an acid (e.g., acetic acid) or a base (e.g., triethylamine) or under neutral conditions, to provide a carboxylic acid.

The term acid-sensitive ester refers to ester which can be selectively cleaved by treatment with a sufficiently strong acid under mild conditions without affecting other functional groups. Such esters e.g., are tertiary butyl, methylthiomethyl, tetrahydropyranyl, tetrahydrofuranyl, methoxyethoxymethyl, benzyloxymethyl, 1-methyl-1-phenylethyl, 3-methyl-3-pentyl, dicyclopropylmethyl, diphenylmethyl. Additional guidance can be found in e.g. J. F. W. McOmie, "Protective Groups in Organic Chemistry", Plenum Press, London and New York 1973; in T. W. Greene and P. G. M. Wuts, "Greene's Protective Groups in Organic Synthesis", Fourth Edition, Wiley, New York 2007.

Sufficiently strong acids means that they are able to essentially quantitatively protonate the secondary amine in elagolix (and related compounds, respectively) and mediate ester cleavage at a reasonable rate. Such acids are are characterized by a pKa value of <4, preferably are characterized by a pKa value of <3, most preferably are characterized by a pKa value <2.

The pKa as used herein refers to the negative logarithmic of the dissociation constant Ka for an acid in water at 20°C.

The term "one-pot" "or "one-pot process" means that in a series (i.e. in a succession) of reactions, for example two or more successive reactions, each reaction product is provided for the next reaction without isolation and purification. The one-pot processes defined herein encompass not only a series (i.e. a succession) of reactions conducted in a single reaction vessel, but also a series (i.e. a succession) of reactions conducted in a plurality of reaction vessels (e.g., by transferring the reaction mixture from one vessel to other) without isolation and purification. Preferably, the one-pot process is conducted in a single reaction vessel.

The term "step-wise process" means that in a series (i.e a succession) of reactions, each reaction product is provided for the next reaction with isolation and optionally purification.

The term "work-up" means the work of isolation and/or purification which is carried out once the reaction is finished.

As used herein, unless specified otherwise, the term "room temperature" or "ambient temperature" means a temperature of from 15 to 30°C, such as from 20 to 30°C, such as from 20 to 25°C.

The term "inert" as used throughout this application, means unreactive with any of the reactants, solvents, or other components of the reaction mixture. Such inert conditions are generally accomplished by using inert gas such as carbon dioxide, helium, nitrogen, argon, among other gases.

The compounds of the present invention can possess one or more asymmetric centers. The preferred absolute configurations are as indicated herein specifically. However, any possible pure enantiomer, pure diastereoisomer, or mixtures thereof, e.g., mixtures of enantiomers, such as racemates, are encompassed by the present invention.
In the formulae of the present application the term " 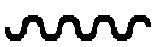 ", " 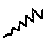 " or " 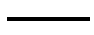 " on a C-sp3 represents a covalent bond wherein the stereochemistry of the bond is not defined. This means that the term " 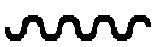 " or " 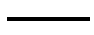 " on a C-sp3 comprises an (S) configuration as well as an (R) configuration of the respective chiral centre. Furthermore, mixtures are also encompassed, e.g., mixtures of enantiomers, such as racemates, are encompassed by the present invention.

In the formulae of the present application the term " 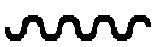 " or " 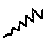 " on a C-sp2 represents a covalent bond, wherein the stereochemistry or the geometry of the bond is not defined. This means that the term " 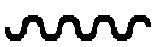 " on a C-sp2 comprises a cis (Z) configuration as well as a trans (E) configuration of the respective double bond. Furthermore, mixtures are also encompassed, e.g., mixtures of double bond isomers are encompassed by the present invention.
In the formulae of the present application, the term " 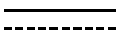 " indicates a Csp3-Csp3 bond or a Csp2-Csp2 bond.

The compounds of the present invention can possess one or more asymmetric centers. The preferred absolute configurations are as indicated herein specifically.
In the formulae of the present application the term " 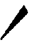 " on a C-sp3 indicates the absolute stereochemistry, either (R) or (S).
In the formulae of the present application the term " 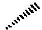 " on a C-sp3 indicates the absolute stereochemistry, either (R) or (S).

Terms d,l and meso are used herein following stereodescriptor nomenclature according to: Gutsche, C. D.; Pasto, D. J. Fundamentals of Organic Chemistry, Prentice-Hall, Inc., Englewood Cliffs, New Jersey, 1975 and, Eliel, E. L.; Wilen, S. H. Stereochemistry of Organic Compounds, John Wiley & Sons, Inc. 1994.

The term "stereomeric purity" at a given percentage means that the designated stereoisomer predominates at that given percentage in a mixture of stereoisomers.

The term "tautomer" refers in particular to the aldehyde tautomer of compounds of the present invention, where such compounds can exists in either an enol or aldehyde form, or mixtures thereof.

Salts are especially pharmaceutically acceptable salts or generally salts of any of the intermediates mentioned herein, where salts are not excluded for chemical reasons the skilled person will readily understand. They can be formed where salt forming groups, such as basic or acidic groups, are present that can exist in dissociated form at least partially, e.g. in a pH range from 4 to 10 in aqueous solutions, or can be isolated especially in solid, especially crystalline, form.
Such salts are formed, for example, as acid addition salts, preferably with organic or inorganic acids, from compounds or any of the intermediates mentioned herein with a basic nitrogen atom (e.g. imino or amino), especially the pharmaceutically acceptable salts.

Suitable inorganic acids are acids having a pKa of at most 4.0, for example, halogen acids such as hydrochloric acid or sulfuric acid. Suitable organic acids are acids having a pKa of at most 4.0, for example, certain carboxylic, phosphonic, sulfonic or sulfamic acids. For example methane- or ethane-sulfonic acid, ethane-1,2-disulfonic acid, benzenesulfonic acid, 2-naphthalenesulfonic acid, 1,5-naphthalene-disulfonic acid, formic acid or trifluoroacetic acid.

When a basic group and an acid group are present in the same molecule, any of the intermediates mentioned herein may also form internal salts.

In view of the close relationship between the compounds and intermediates in free form and in the form of their salts, including those salts that can be used as intermediates, for example in the purification or identification of the compounds or salts thereof, any reference to "compounds", "starting materials" and "intermediates" hereinbefore and hereinafter is to be understood as referring also to one or more salts thereof or a mixture of a corresponding free compound, intermediate or starting material and one or more salts thereof, each of which is intended to include also any solvate or salt of any one or more of these, as appropriate and expedient and if not explicitly mentioned otherwise. Different crystal forms may be obtainable and then are also included.

Where the plural form is used for compounds, starting materials, intermediates, salts, pharmaceutical preparations, diseases, disorders and the like, this is intended to mean one (preferred) or more single compound(s), salt(s), pharmaceutical preparation(s), disease(s), disorder(s) or the like, where the singular or the indefinite article ("a", "an") is used, this is not intended to exclude the plural, but only preferably means "one".

The term "substituted" preferably means that 1-3 substituents are present, which are independently selected from halogen, -OH, -COOH, C₁₋₆ alkyl, C₁₋₆ haloalkyl, -N(C₁₋₆ alkyl)₂, -NH(C₁₋₆ alkyl), =O, CN, C₁₋₆ alkoxy and NH₂.

The term "substituted" most preferably means that 1-3 substituents are present, which are independently selected from halogen, C₁₋₆ alkyl, C₁₋₆ haloalkyl, -N(C₁₋₆ alkyl)₂, CN and C₁₋₆ alkoxy.

### Abbreviations:

- µm: micrometre
- µL: microlitre
- aq: aqueous
- ar: aromatic
- arom: aromatic
- Ac: acetyl
- AcOH: acetic acid
- Boc: tert-butoxycarbonyl
- CO2: carbon dioxide
- Cy: cyclohexyl

- DABCO: 1,4-diazobicyclo[2.2.2]octane
- DBU: 1,8-diazabicyclo[5,4,0]undec-7-ene
- DCM: dichloromethane
- DIEA: N-ethyldiisopropylamine
- DME: 1,2-dimethoxyethane
- DMF = dmf: dimethylformamide
- DMPU: 1,3-dimethyl-3,4,5,6-tetrahedrao-2(1H)-pyrimidinone
- DMSO: dimethylsulfoxide
- DMSO-d6: dimethylsulfoxide deuterated

- ee: enantiomeric excess
- equiv: equivalent
- ES: electrospray
- ES+: positive electrospray ionisation
- ESI: electrospray ionisation
- Et: ethyl
- Et2O: diethyl ether
- EtOAc: ethyl acetate
- EtOH: ethanol

- g: gram(s)
- GC: gass chromatography

- h: hour(s)
- HCl: hydrochloric acid
- HCl(aq): hydrogen chloride aqueous solution
- 1HNMR: proton nuclear magnetic resonance
- HPLC: high performance liquid chromatography
- H3PO4: phosphoric acid
- HRMS: high resolution mass spectroscopy
- H2SO4: sulfuric acid
- Hz: hertz

- iPr: isopropyl
- iPrOAc: isopropyl acetate
- iPrOH: isopropanol
- IR: infrared

- J: coupling constant

- K2CO3: potassium carbonate

- L: litre
- LC-MS: liquid chromatography-mass spectrometry
- LCMS: liquid chromatography-mass spectrometry
- LRMS: low resolution mass spectroscopy

- m/e: mass-to-charge ratio
- mg: milligram
- min: minute(s)
- mL: millilitre
- mmol(s): millimole(s)
- mol(s): mole(s)
- monosub.: monosubstituted
- mp: melting point
- m.p.: melting point
- m/z: mass-to-charge ratio
- M: molarity/molar
- Me: methyl
- 2-MeTHF: 2-methyltetrahydrofuran
- Mel: methyl iodide
- MeOH: methanol
- MeONa: sodium methoxide
- MgSO4: magnesium sulfate
- MS: mass spectrometry
- MTBE: tertbutylmethylether

- nm: nanometre
- N: nitrogen atom
- N2: nitrogen
- NaCl: sodium chloride
- Na2CO3: sodium carbonate
- NaHCO3: sodium bicarbonate
- NaOH: sodium hydroxide
- NaOMe: sodium methoxide
- NH4Cl: ammonium chloride
- NMR: nuclear magnetic resonance

- ppm: parts per million

- RT = rt: room temperature

- temp.: temperature
- T: temperature
- tR: retention time
- tert-butyl: tertiary-butyl
- TEA: triethylamine
- TFA: trifluoroacetic acid
- THF: tetrahydrofuran
- TLC: thin layer chromatography
- UV: ultraviolet light
- vol.: volume
- wt.: weight
- Xyl: xylene

### Literature references for chemical processes/products

Standard conditions for group transformations are described, for example, in the relevant chapters in Richard C. Larock, "Comprehensive Organic Transformations: A Guide to Functional Group Preparations", Second Edition, Wiley-VCH Verlag GmbH, 2000.

General reactions and mechanisms in organic chemistry are described, for example, in Smith, M., B.; March, J.; March's Advanced Organic Chemistry: Reactions, Mechanisms and Structure, 6th Edition, John Wiley & Sons, Inc., 2007; in particular as described in the relevant chapters thereof;

Protecting groups are described, for example, in
J. F. W. McOmie, "Protective Groups in Organic Chemistry", Plenum Press, London and New York 1973; in T. W. Greene and P. G. M. Wuts, "Greene's Protective Groups in Organic Synthesis", Fourth Edition, Wiley, New York 2007; in "The Peptides"; Volume 3 (editors: E. Gross and J. Meienhofer), Academic Press, London and New York 1981, and in "Methoden der organischen Chemie" (Methods of Or-ganic Chemistry), Houben-Weyl, 4th edition, Volume 15/l, Georg Thieme Verlag, Stuttgart 1974.

Pharmaceutical Salts are described, for example, in
Handbook of Pharmaceutical Salts: Properties, Selection, and Use Edited by P. Heinrich Stahl and Camile G. Wermuth. VHCA, Verlag Helvetica Chimica Acta, Zurich, Switzerland, and Wiley-VCH, Weinheim, Germany. 2002.

### BRIEF DESCRIPTION OF THE DRAWINGS

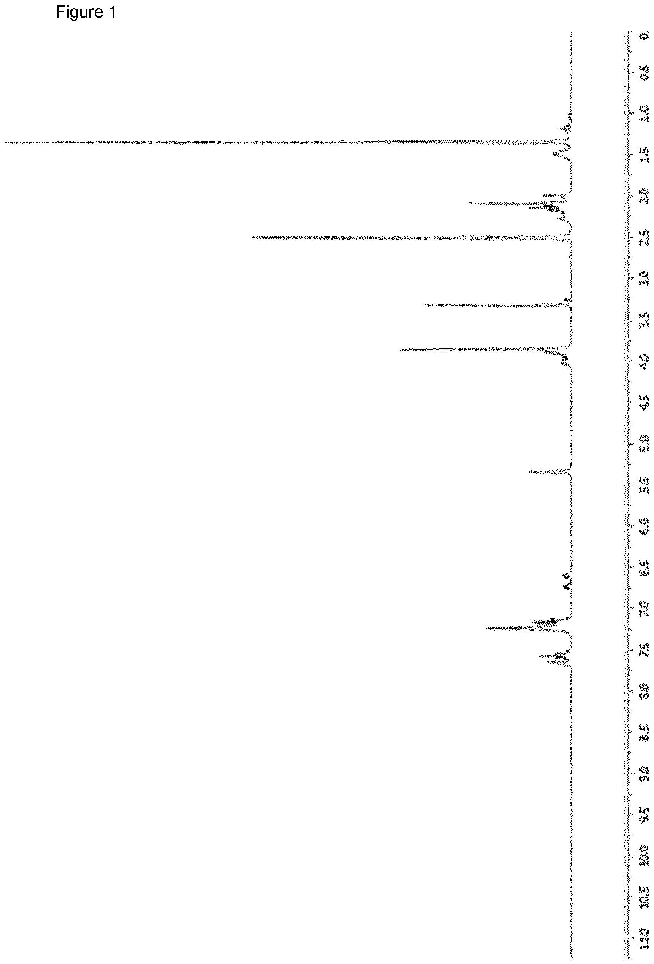

FIG. 1: 1H NMR of tert-butyl (R)-4-((2-(5-(2-fluoro-3-methoxyphenyl)-3-(2-fluoro-6-(trifluoromethyl)benzyl)-4-methyl-2,6-dioxo-3,6-dihydropyrimidin-1(2H)-yl)-1-phenylethyl)-amino)butanoate (tert-butyl ester of elagolix) in d6-DMSO obtained from Example 1.
FIG. 2: illustrates a representative PXRD of the crystalline acid addition salt of elagolix with sulfuric acid of the present invention. The x-axis shows the scattering angle in °2-Theta, the y-axis shows the intensity of the scattered X-ray beam in counts of detected photons.
FIG. 3: illustrates a representative PXRD of the amorphous acid addition salt of elagolix with hydrochloric acid of the present invention. The x-axis shows the scattering angle in °2-Theta, the y-axis shows the intensity of the scattered X-ray beam in counts of detected photons.

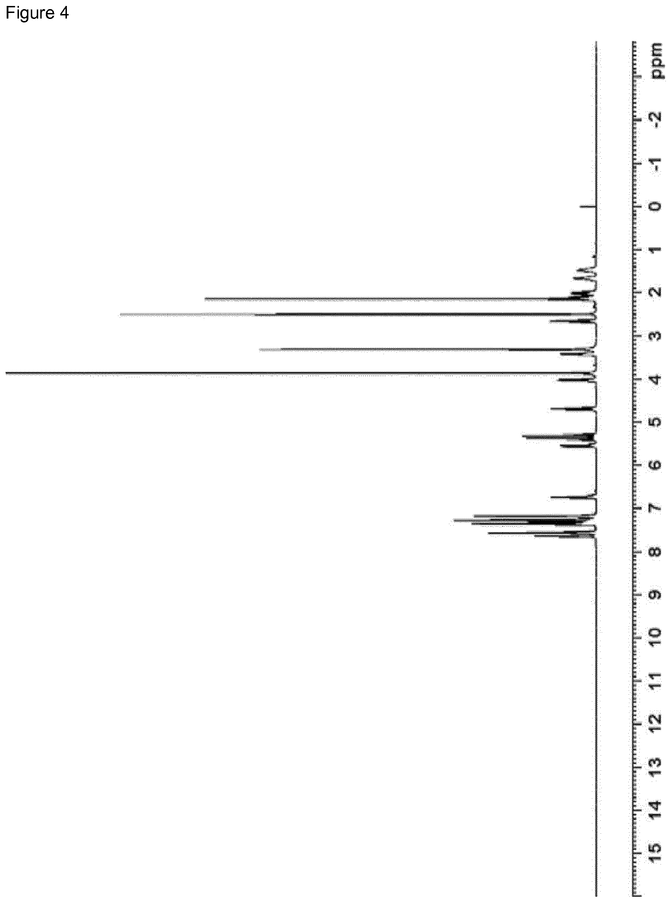

FIG. 4: 1H NMR of (R)-5-(2-fluoro-3-methoxyphenyl)-1-(2-fluoro-6-(trifluoromethyl)benzyl)-6-methyl-3-(2-(2-oxopyrrolidin-1-yl)-2-phenylethyl)pyrimidine-2,4(1H,3H)-dione in d6-DMSO obtained from Example 4.
FIG. 5: illustrates a representative PXRD of (R)-5-(2-fluoro-3-methoxyphenyl)-1-(2-fluoro-6-(trifluoromethyl)benzyl)-6-methyl-3-(2-(2-oxopyrrolidin-1-yl)-2-phenylethyl)pyrimidine-2,4(1H,3H)-dione in d6-DMSO obtained from Example 4. The x-axis shows the scattering angle in °2-Theta, the y-axis shows the intensity of the scattered X-ray beam in counts of detected photons.

### DETAILED DESCRIPTION OF THE INVENTION

The present invention relates to a process starting from an acid sensitive ester of Formula (3) or Formula (III) or Formula (IIIa) comprising:
a) dissolve an acid-sensitive ester of Formula (3), or Formula (III), or Formula (IIIa) in a solvent
b) add an acid sufficiently strong to cleave the acid-sensitive ester
c) optionally, add an anti-solvent
d) isolate the solid acid addition salt of elagolix by filtration
e) optionally, dry the acid addition salt of Formula (2), or Formula (II) or Formula (IIa).

The invention also refers to the following aspects:
A process for the preparation of an acid addition salt of elagolix comprising
a) dissolve the t-butyl ester of elagolix of Formula (IIIaa) in a solvent
b) add an acid sufficiently strong to cleave the acid-sensitive ester
c) optionally, add an anti-solvent
d) isolate the solid acid addition salt of elagolix by filtration
e) optionally, dry the acid addition salt of elagolix.

A process of the invention wherein the solvent is selected from isoropanol, ethanol, acetonitrile, EtOAc, THF, dioxane or mixtures thereof, preferably, isopropanol, EtOAc or mixtures thereof.

A process of the invention wherein the acid is selected from HCl, H2SO4, TFA or HCOOH, preferably, HCl or H2SO4.

A process of the invention wherein optionally an anti-solvent selected from H2O, aliphatic or aromatic hydrocarbons, preferably, H2O, heptane, cyclohexane.

The process is performed at ambient temperature or elevated temperature in a range of 30 °C to 70 °C, preferably, the process is performed at ambient temperature.

The amount of the acid used ranges from 1.1 to 10, equivalent, preferable from 2 to 5 equivalents.The skilled person will appreciate that a slight molar excess of acid is best used to fully protonate the secondary amine and then also acid-catalyze ester hydrolysis.

The acid-sensitive ester of elagolix can be prepared according processes described in WO2005/007165 (e.g., Step 7I of Example 7) and WO2009/062087 (e.g., Example 4, Step 4A) starting from the primary amine (Formula IV) comprising
a) Reacting the primary amine of Formula (IVa) (or the related compounds of Formula (4), or of Formula (IV) in a solvent with an acid-sensitive ester of a 4-halobutyric acid, in the presence of amine
b) Optionally, isolate the acid sensitive ester of elagolix or a related compound (Formula IIIa/III/3).

More specifically the tert-butyl ester of elagolix (Formula IIIaa) can be prepared comprising:
a) Reacting the primary amine of Formula (IVa) in in a solvent with tert-butyl 4-bromobutyrate, in the presence of an amine, preferentially K₂CO₃, Na₂CO₃, DIEA or TEA
b) Optionally, isolate the acid-sensitive tert-butyl ester of elagolix (Formula IIIaa).

The process for the preparation of the tert-butyl ester (Formula IIIaa) is summarized in Scheme1.

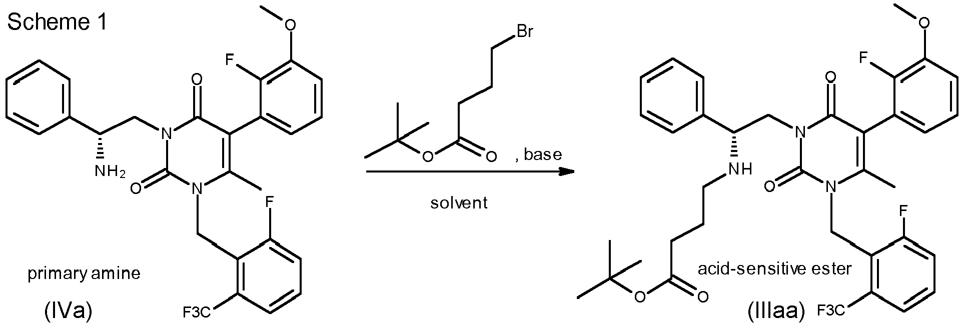

The process wherein the solvent is selected from DMF, DMSO, DMPU or acetonitrile, preferably solvent is DMF or acetonitrile.

The process wherein the base is selected from K₂CO₃, Na₂CO₃, DIEA or TEA, preferably, the base is K2CO3 or DIEA.

The process is preferably performed at ambient temperature or elevated temperature in a range of 40 °C to 70 °C, preferably, the reaction is performed between 40 and 65 °C.

Alternatively, the acid sensitive ester can be prepared by reaction of an amine of Formula (4) or (IV) or (IVa) with an acid-sensitive ester of 4-oxobutanoic acid in the presence of an reductive agent such as e.g., NaBH3CN, Na(OAc)3BH or borane pyridine complex.

### General Process Conditions

The following, in accordance with the knowledge of a person skilled in the art about possible limitations in the case of single reactions, applies in general to all processes mentioned hereinbefore and hereinafter, while reaction conditions specifically mentioned above or below are preferred.

In any of the reactions mentioned hereinbefore and hereinafter, protecting groups may be used where appropriate or desired, even if this is not mentioned specifically, to protect functional groups that are not intended to take part in a given reaction, and they can be introduced and/or removed at appropriate or desired stages. Reactions comprising the use of protecting groups are therefore included as possible wherever reactions without specific mentioning of protection and/or deprotection are described in this specification. However, reactions without protecting groups are preferred in the context of the present invention.

Within the scope of this disclosure only a readily removable group that is not a constituent of the particular desired end product is designated a "protecting group", unless the context indicates otherwise. The protection of functional groups by such protecting groups, the protecting groups themselves, and the reactions appropriate for their introduction and removal are described for example in standard reference works, such as J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/l, Georg Thieme Verlag, Stuttgart 1974, in H.-D. Jakubke and H. Jesch-keit, "Aminosäuren, Peptide, Proteine" (Amino acids, Peptides, Proteins), Verlag Chemie, Weinheim, Deerfield Beach, and Basel 1982, in "Protecting Groups", Philip J. Kocienski, 3rd Edition, GeorgThieme Verlag, Stuttgart, ISBN 3-13-137003-3 and in Jochen Lehmann, "Chemie der Kohlenhydrate: Monosaccharide und Derivate" (Chemistry of Carbohydrates: Monosaccharides and Derivatives), Georg Thieme Verlag, Stuttgart 1974, in particular in the relevant chapters thereof. A characteristic of protecting groups is that they can be removed readily (i.e. without the occurrence of undesired secondary reactions) for example by solvolysis, reduction, photolysis or alternatively under physiological conditions (e.g., by enzymatic cleavage). Different protecting groups can be selected so that they can be removed selectively at different steps while other protecting groups remain intact. The corresponding alternatives can be selected readily by the person skilled in the art from those given in the standard reference works mentioned above or the description or the Examples given herein.

All the above-mentioned process steps can be carried out under standard reaction conditions known in the art, unless otherwise specified, preferably those mentioned specifically, in the absence or, customarily, in the presence of solvents or diluents, preferably solvents or diluents that are inert towards the reagents used and dissolve them, in the absence or presence of catalysts, condensation or neutralizing agents, for example ion exchangers, such as cation exchangers, e.g. in the H+ form, depending on the nature of the reaction and/or of the reactants at reduced, normal or elevated temperature, for example in a temperature range of from about -100 °C to about 190 °C, preferably from approximately -80 °C to approximately 150 °C, for example at from -80 to -60°C, at room temperature, at from -20 to 40 °C or at reflux temperature, under atmospheric pressure or in a closed vessel, where appropriate under pressure, and/or in an inert atmosphere, for example under an argon or nitrogen atmosphere.

Where required, the working-up of reaction mixtures, especially in order to isolate desired compounds or intermediates, follows customary procedures and steps, e.g. selected from the group comprising but not limited to extraction, neutralization, crystallization, chromatography, evaporation, drying, filtration, centrifugation and the like.

The invention relates also to those forms of the process in which a compound obtainable as intermediate at any stage of the process is used as starting material and the remaining process steps are carried out, or in which a starting material is formed under the reaction conditions or is used in the form of a derivative, for example in protected form or in the form of a salt, or a compound obtainable by the process according to the invention is produced under the process conditions and processed further in situ. In the process of the present invention those starting materials are preferably used which result in compounds of formula 1/I/Ia which are described as being preferred. Special preference is given to reaction conditions that are identical or analogous to those mentioned in the Examples. The invention relates also to novel starting compounds and intermediates described herein, especially those leading to compounds mentioned as preferred herein.

The invention especially relates to any of the methods described hereinbefore and hereinafter that leads to the product, or a pharmaceutically acceptable salt thereof.

The following Examples serve to illustrate the invention without limiting the scope thereof, while they on the other hand represent preferred embodiments of the reaction steps, intermediates and/or the process of manufacture of the product in free base form or as a pharmaceutically acceptable salt thereof.

### Embodiment Section

1. Process for the preparation of an acid addition salt of Formula (2) 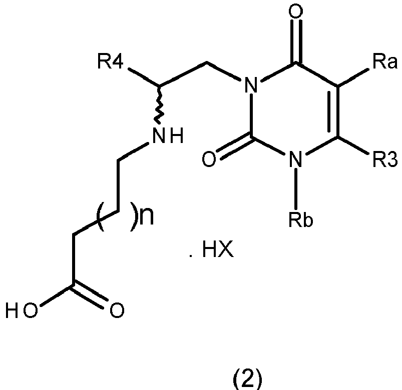 preferably 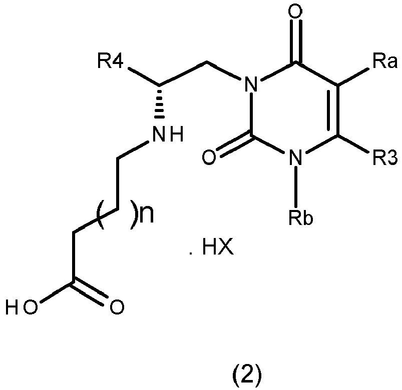 wherein
   n = 1 or 2;
   Rₐ and R_{b} are independently of each other a substituted or unsubstituted C₁₋₂₀ alkyl, substituted or unsubstituted C₁₋₂₀ alkoxy, substituted or unsubstituted C₁₋₂₀ alkenyl, substituted or unsubstituted C₃₋₁₂ cycloalkyl, substituted or unsubstituted C₆₋₁₂ aryl, substituted or unsubstituted C₅₋₁₂ heteroaryl, substituted or unsubstituted C₇₋₁₂ alkylheteroaryl, substituted or unsubstituted C₇₋₁₂ alkylaryl, substituted or unsubstituted C₃₋₁₂ heterocycloalkyl, substituted or unsubstituted C₅₋₁₂ heterobicycle, substituted or unsubstituted C₅₋₁₂ biaryl, substituted or unsubstituted C₅₋₁₂ bicycloaryl, substituted or unsubstituted C₅₋₁₂ bicycloalkyl, substituted or unsubstituted -C₃₋₇ cycloalkyl-C₃₋₇ heterocycle, substituted or unsubstituted -C₃₋₇ aryl-C₃₋₇ heterocycle, or substituted or unsubstituted -C₃₋₇ heterocycloalkyl-C₃₋₇ heteroaryl;
   R₃ = hydrogen or methyl;
   R₄ = substituted or unsubstituted phenyl, substituted or unsubstituted C₃₋₇ alkyl, hydrogen, -C₁₋₆ alkanediyl-COOH, or -C(=O)O-(t-butyl);
   HX = organic or inorganic acid;
   preferably of Formula (II) 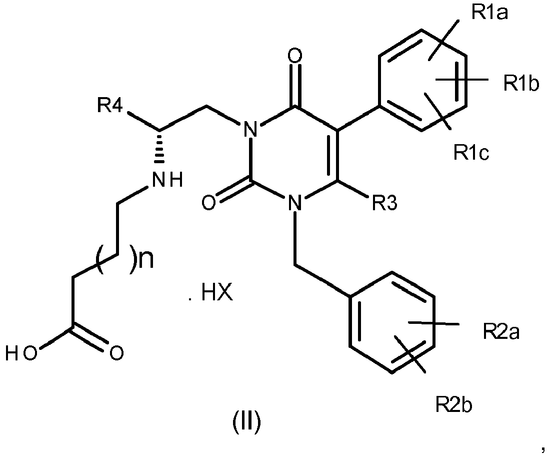 wherein
   n = 1 or 2, preferably n = 1:
   R₁ₐ, R_{1b} and R_{1c} are independently selected from hydrogen, halogen, substituted or unsubstituted C₁₋₄ alkyl, hydroxy, substituted or unsubstituted C₁₋₄ alkoxy, or R₁ₐ, and R_{1b} when taken together form -OCH₂O- or -OCH₂CH₂-; preferably, R₁ₐ, R_{1b} and R_{1c} are independently selected from hydrogen, halogen, unsubstituted C₁₋₄ alkyl, hydroxy, and unsubstituted C₁₋₄ alkoxy;
   R₂ₐ, and R_{2b} are independently selected from hydrogen, halogen, trifluoromethyl, cyano, or -SO₂CH₃;
   R₃ = hydrogen or methyl;
   R₄ = substituted or unsubstituted phenyl, substituted or unsubstituted C₃₋₇ alkyl, hydrogen, -C₁₋₆-alkanediyl-COOH, or -C(=O)O-(t-butyl);
   HX = (sufficiently strong) organic or inorganic acid;
   comprising the steps of:
      a) dissolving an (acid sensitive) ester of Formula (3), preferably of Formula (III) 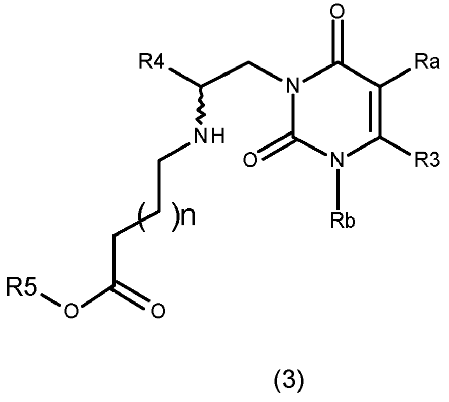 preferably 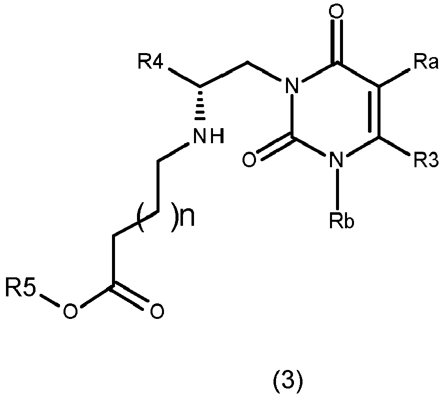 wherein n, Rₐ, R_{b}, R₃, and R₄ are as defined above; and
         wherein R₅ is a protective group, 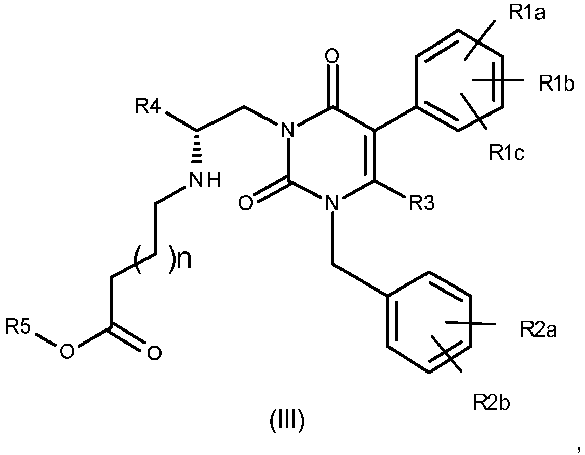 wherein n, R₁ₐ, R_{1b}, R_{1c}, R₂ₐ, R_{2b}, R₃, and R₄ are as defined above; and
         wherein R₅ is a protective group,
         in a solvent;
      b) adding an organic or inorganic acid to cleave the ester of Formula (3) or Formula (III)
      c) optionally, adding an anti-solvent;
      d) isolating a solid acid addition salt of Formula (2) or Formula (II) by filtration; and
      e) optionally, drying the solid acid addition salt of Formula (2) or Formula (II).
2. The process of item 1, wherein the protection group R₅ in Formula (3) or Formula (III) is selected from the group consisting of tert-butyl-, triphenylmethyl-, and trialkylsilyl- (e.g., tri(C₁₋₆alkyl)silyl-, for example trimethylsilyl-).
3. The process of item 1 or 2, wherein the substituents in Formula (II) are defined as follows:
   n = 1;
   R₁ₐ, = halogen, preferably fluorine;
   R_{1b} = unsubstituted C₁₋₄ alkoxy;
   R_{1c} = hydrogen;
   R₂ₐ, and R_{2b} are independently selected from hydrogen, and halogen;
   R₃ = hydrogen or methyl;
   R₄ = unsubstituted phenyl or phenyl substituted with halogen, or unsubstituted C₃₋₇ alkyl; preferably unsubstituted phenyl, or unsubstituted C₃₋₇ alkyl;
   HX = organic or inorganic acid, preferably HCl, or H₂SO₄.
4. The process of any one of the preceding items, wherein the substituents in Formula (II) are defined as follows:
   n = 1;
   R₁ₐ, = halogen,
   R_{1b} = unsubstituted C₁₋₄ alkoxy
   R_{1c} = hydrogen;
   R₂ₐ, and R_{2b} are independently selected from hydrogen, and halogen;
   R₃ = hydrogen or methyl;
   R₄ = unsubstituted phenyl;
   HX = organic or inorganic acid, preferably HCI, or H₂SO₄.
5. The process of item 1 or 2, wherein the acid addition salt of Formula (II) is an acid addition salt of elagolix and has the Formula (IIa) 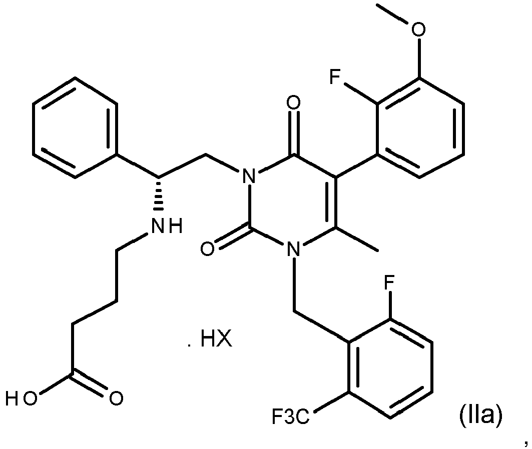 and
   wherein the (acid sensitive) ester of Formula (III) is an ester of elagolix and has the Formula (IIIa) 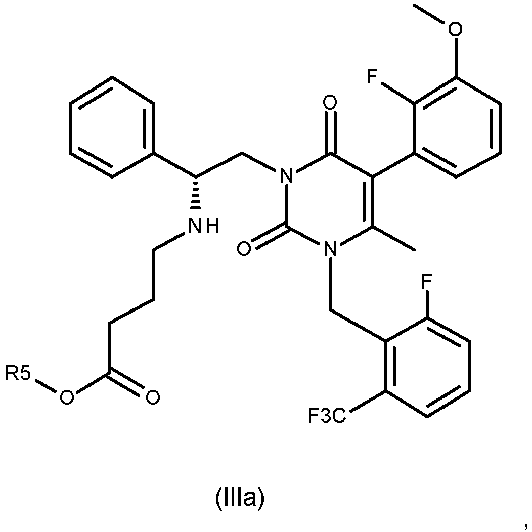 wherein R₅ is as defined in item 1 or 2.
6. The process of any of the preceding items, wherein the solvent is isopropanol, ethylacetate (EtOAc) or mixtures thereof.
7. The process of any of the preceding items, wherein the (sufficiently strong) acid is selected from HCI, trifluoroacetic acid (TFA), HCOOH, H₂SO₄ and mixtures thereof.
8. The process of any of the preceding items, wherein the ester of Formula (III), preferably (IIIa), is prepared by a process comprising the steps of:
   aa) reacting the primary amine of the compound of Formula (4), preferably Formula (IV), further preferred of Formula (IVa) 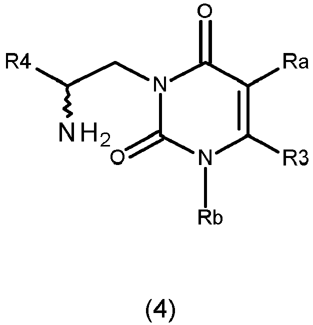 preferably 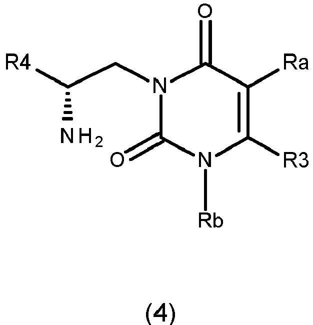 wherein Rₐ, R_{b}, R₃, and R₄ are as defined above; 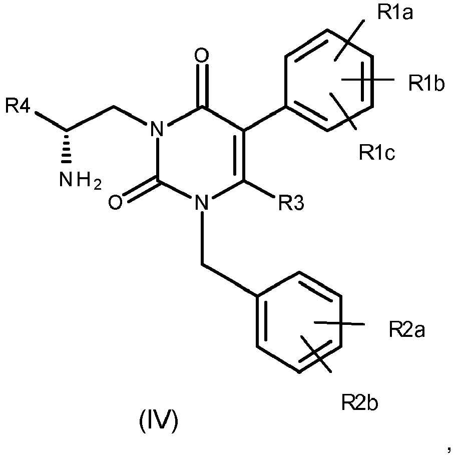 wherein R₁ₐ, R_{1b}, R_{1c}, R₂ₐ, R_{2b}, R₃, and R₄ are as defined in any of the preceding items; 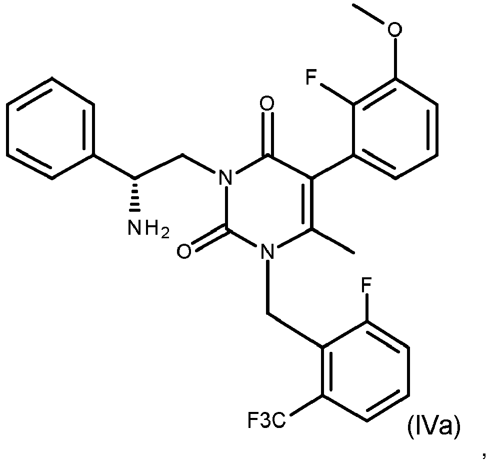 with an ester of 4-halobutyric acid, preferably tert-butyl 4-bromobutyrate, in the presence of a base, preferably K₂CO₃, Na₂CO₃, diisopropylamine (DIEA) or triethylamine (TEA) to provide the compound of Formula (3), preferably Formula (III), further preferred of Formula (IIIa) (at elevated temperature), 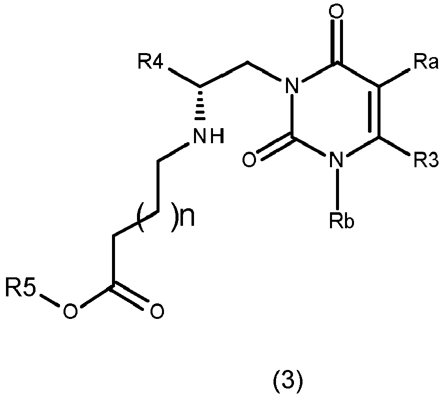 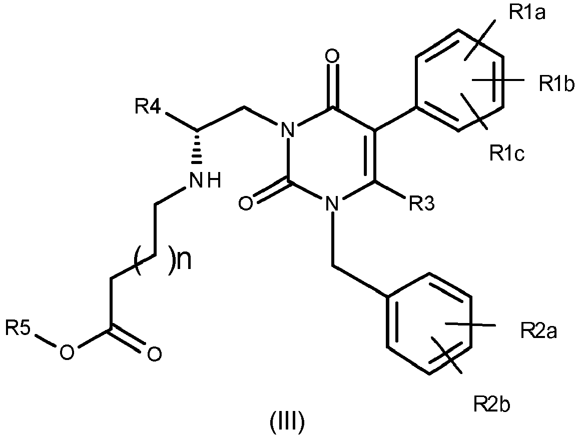 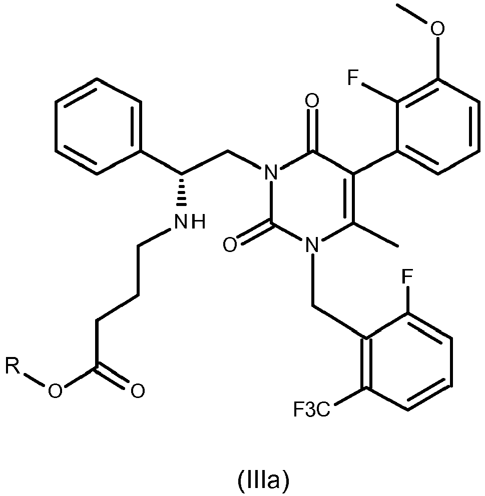
   bb) optionally, isolating the ester of Formula (3)/(III), preferably of Formula (IIIa).
9. The process of any of one of items 1 to 7, wherein an acid addition salt of elagolix is isolated, which is an amorphous or crystalline hydrogensulfate salt, or amorphous or crystalline hydrochloride salt.
10. Acid addition salt of Formula (2), of Formula (II) or Formula (IIa) as defined in any of the preceding items, wherein HX is HCI, and H₂SO₄.
11. Acid addition salt of Formula (2), of Formula (II) or Formula (IIa) as defined in any of the preceding items,
   wherein the acid addition salt of Formula (2) contains an impurity according to Formula (5) 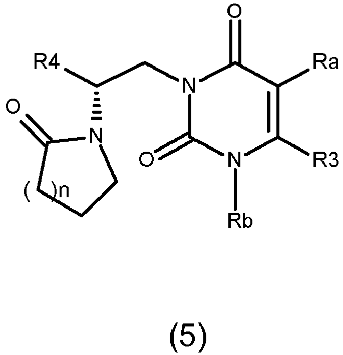 wherein Rₐ, R_{b}, R₃, and R₄ are as defined above;
   wherein the acid addition salt of Formula (II) contains an impurity according to Formula (V) 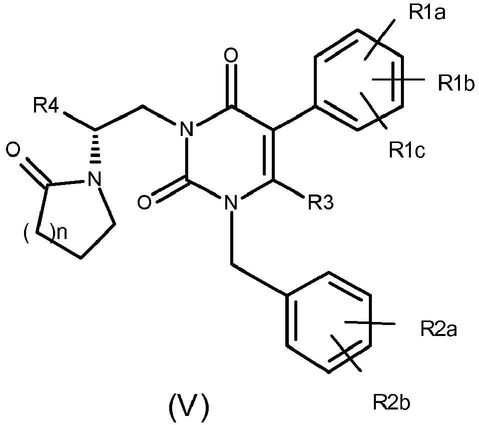 wherein R₁ₐ, R_{1b}, R_{1c}, R₂ₐ, R_{2b}, R₃ and R₄ are as defined for Formula (II) and the acid addition salt of Formula (IIa) contains an impurity according to Formula (Va), respectively, 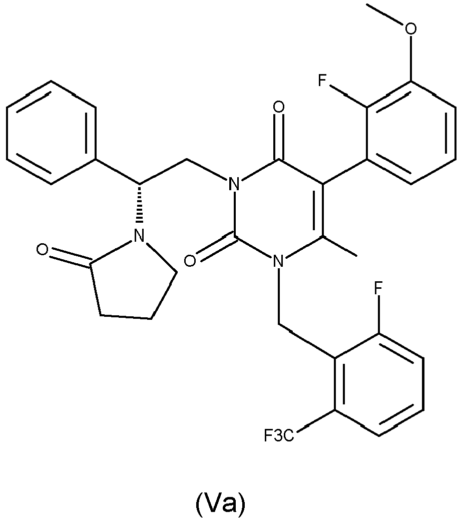 in an amount of at most 1% by weight, for example 0.0 to 1.0% by weight, or for example 0.001 to 0.50% by weight.
12. Pharmaceutical composition comprising an acid addition salt of item 10 or 11 and a pharmaceutically acceptable carrier.
13. Pharmaceutical composition of item 12 for use as medicament.
14. Pharmaceutical composition of item 12 for use in a method of treatment or prevention of endometriosis and uterine fibroids.
15. The process of any of items 1-9, wherein the acid-sensitive ester is a tert-butyl ester of Formula (IIIaa) 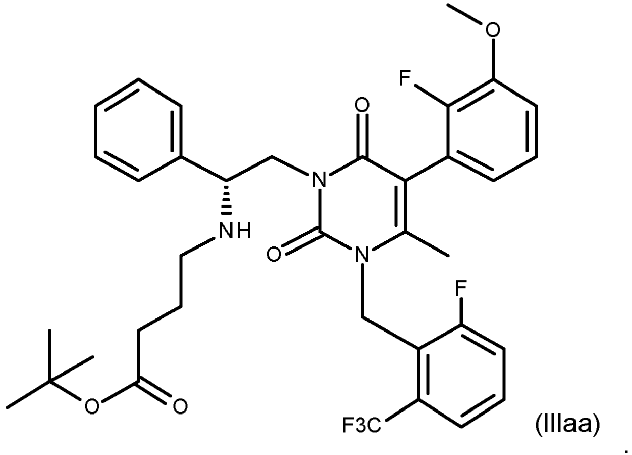
16. The process of any of items 1 to 9 and 15, wherein the acid is HCI, or H₂SO₄, preferably H₂SO₄.
17. The process of any of items 1 to 9 and 15-16, wherein an anti-solvent is added, preferably wherein the anti-solvent is water.
18. The process of any of items 1 to 9 and 15-17, wherein the anti-solvent is an aliphatic or aromatic hydrocarbon, preferably heptane or cyclohexane.
19. The process of any of items 1 to 9 and 15-18, wherein the primary amine of Formula (4), Formula (IV) or (IVa) is reacted with an acid-sensitive ester of a 4-halobutyric acid, in the presence of amine, preferentially K₂CO₃, Na₂CO₃, DIEA or TEA at preferably elevated temperature (e.g., above 30°C, or above 50°C).
20. The process of any of items 1 to 9 and 15-19, wherein the ester of Formula (4), of Formula (III) and Formula (IIIa), respectively, is prepared by reductive amination of the compound of Formula (IV) and Formula (IVa), respectively, with an acid sensitive ester of 4-oxobutanoic acid in the presence of a reducing agent.
21. The process of any of items 1 to 9 and 15-20, wherein the isolated acid addition salt of Formula (2), Formula (II) and Formula (IIa), respectively, is dried under reduced pressure.
22. The process of any of items 1 to 9 and 15-21, wherein the isolated acid addition salt of Formula (2), of Formula (II) and Formula (IIa), respectively, is amorphous.
23. The process of any of items 1 to 9 and 15-22, wherein the isolated wherein the isolated acid addition salt of Formula (2), of Formula (II) and Formula (IIa), respectively, is the amorphous hydrochloride salt.
24. The process of any of items 1 to 9 and 15-22, wherein the isolated wherein the isolated acid addition salt of Formula (2), of Formula (II) and Formula (IIa), respectively, is the amorphous hydrogensulfate salt.
25. The process of any of items 1 to 9 and 15-21, wherein the isolated wherein the isolated acid addition salt of Formula (2), of Formula (II) and Formula (IIa), respectively, is crystalline.
26. The process of any of items 1 to 9 and 15-21, wherein the isolated wherein the isolated acid addition salt of Formula (2), of Formula (II) and Formula (IIa), respectively, is a crystalline hydrogensulfate salt.
27. Use of the ester of Formula (3), of Formula (III) and Formula (IIIa), respectively, for the preparation of an acid addition salt of of Formula (2), Formula (II) or (IIa), preferably by using the process as defined in any of items 1-9 and 15-26.
28. Pharmaceutical compositions comprising an acid addition salt of Formula (2), of Formula (II) and Formula (IIa), respectively, prepared by a process of any of one of items 1 to 9 and 15-27.
29. Pharmaceutical compositions comprising an amorphous hydrochloride salt of elagolix for Formula (IIa), prepared by a process of any of one of embodiments 5 to 9 and 15 to 23.
30. Pharmaceutical compositions comprising an amorphous hydrogensulfate salt of elagolix of Formula (IIa), prepared by a process of any of one of embodiments 5 to 9 and 15 to 22.
31. Use of pharmaceutical composition of item 12 for the treatment or prevention of endometriosis and uterine fibroids.
32. Method of treating a patient by using the pharmaceutical compositions of the present invention, in particular patients suffering from endometriosis and uterine fibroids.
33. The process, use, pharmaceutical compositions, or method of any preceding items, wherein the acid addition salt is in an about 1:1 stoichiometry of the drug compound and HX.
34. Acid addition salt of Formula (2), Formula (II), or Formula (IIa) of the invention for use as medicament, preferably for use in a method of treatment or prevention of endometriosis and uterine fibroids.
35. The process of any one of items 1-6 or 15, wherein the pKa of the acid added in step (b) is at most 4.0, preferably at most 3.0, more preferably at most 2.0.
36. The acid addition salt of item 11, wherein the conjugated acid of the anion in the acid addition salt has a pKa of at most 4.0, preferably at most 3.0, more preferably at most 2.0.

### Examples

### Example 1: tert-butyl (R)-4-((2-(5-(2-fluoro-3-methoxyphenyl)-3-(2-fluoro-6-(trifluoromethyl)-benzyl)-4-methyl-2,6-dioxo-3,6-dihydropyrimidin-1(2H)-yl)-1-phenylethyl)amino)butanoate (elagolix tert-butyl ester)

(R)-3-(2-amino-2-phenylethyl)-5-(2-fluoro-3-methoxyphenyl)-1-(2-fluoro-6-(trifluoromethyl)-benzyl)-6-methylpyrimidine-2,4(1H,3H)-dione (71.5 g) prepared according to the procedure disclosed in WO 2009/062087 A1, tBu 4-bromobutyrate (35.69 g), and DIPEA (29.7 mL) in a round bottom flask (2L) were dissolved in DMF (358 mL) and stirred at 60°C until reaction was completed. Then the reaction mixture was diluted with ethyl acetate and washed with an aqueous 1M HCI solution. Layers were separated and the organic phase was dried over magnesium sulfate, the solvent was evaporated together with silica gel and purified via column chromatography using hexane/ethyl acetate as an eluent (from 0% to 30%) providing 50.0 g (56.4%) of pure (99.8%) title compound.

### Example 2: Amorphous acid addition salt of elagolix with sulfuric acid from tert-butyl (R)-4-((2-(5-(2-fluoro-3-methoxyphenyl)-3-(2-fluoro-6-(trifluoromethyl)benzyl)-4-methyl-2,6-dioxo-3,6-dihydropyrimidin-1(2H)-yl)-1-phenylethyl)amino)butanoate (hydrogen sulfate salt):

Tert-butyl (R)-4-((2-(5-(2-fluoro-3-methoxyphenyl)-3-(2-fluoro-6-(trifluoromethyl)benzyl)-4-methyl-2,6-dioxo-3,6-dihydropyrimidin-1(2H)-yl)-1-phenylethyl)amino)butanoate (218mg) was dissolved in isopropanol (4 mL) and sulfuric acid (1,88 mL) was added. The resulting mixture was stirred at room temperature for 15 h. Then water was slowly added to obtain a slurry. Amorphous elagolix hydrogen sulfate was then isolated by filtration and dried in vacuo. The acid addition salt is highly pure with a low content of the lactam impurity.

### Reference example 1: Crystalline acid addition salt of elagolix with sulfuric acid (hydrogen sulfate salt)

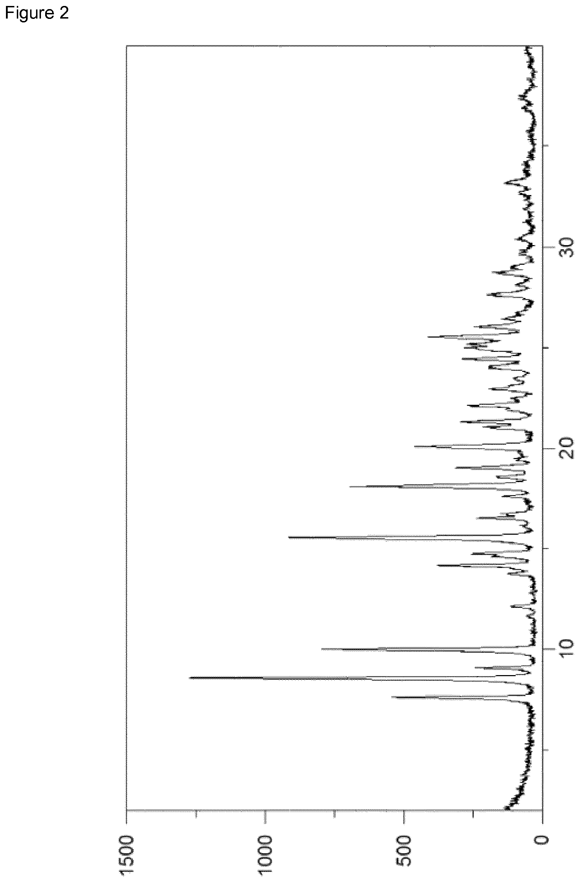

Amorphous elagolix (200 mg, for example prepared according to the procedure disclosed in WO 2009/062087 A1, example 4B) was dissolved in 3.0 mL ethyl acetate, followed by filtration with the aid of a syringe filter (pore size 0.45 microns). Sulfuric acid (95-97 w-% aqueous solution, 1.05 mol equivalent, 18.8 microliter) was diluted with 1.0 mL ethyl acetate and added dropwise to the elagolix solution under stirring. The thus obtained mixture was further stirred at room temperature for 18 hours, leading to the formation of a homogeneous suspension. The solid material was collected by filtration, washed with a little amount of ethyl acetate and dried under vacuum (30 mbar) at room temperature for 16 hours, yielding the acid addition salt of elagolix with sulfuric acid as a white solid. The obtained material was crystalline as confirmed by the PXRD depicted in Figure 2 herein. The corresponding reflection list is provided in Table 1 below:

| Angle [°2-Theta] | Relative Intensity [%] | Angle [°2-Theta] | Relative Intensity [%] |
|---|---|---|---|
| 7.6 | 41 | 21.0 | 13 |
| 8.6 | 100 | 21.3 | 20 |
| 9.1 | 17 | 22.1 | 15 |
| 10.0 | 62 | 22.5 | 5 |
| 11.7 | 2 | 22.9 | 11 |
| 12.1 | 6 | 23.5 | 4 |
| 13.8 | 7 | 24.1 | 11 |
| 14.2 | 28 | 24.4 | 19 |
| 14.8 | 18 | 25.0 | 17 |
| 15.5 | 71 | 25.2 | 17 |
| 16.5 | 16 | 25.5 | 27 |
| 16.7 | 8 | 26.0 | 14 |
| 17.6 | 6 | 26.4 | 7 |
| 18.1 | 48 | 27.6 | 13 |
| 18.6 | 10 | 28.1 | 4 |
| 19.0 | 21 | 28.7 | 9 |
| 19.5 | 3 | 29.0 | 6 |
| 20.1 | 33 | 29.7 | 4 |

### Reference example 2: Amorphous acid addition salt of elagolix with hydrochloric acid (hydrochloride salt)

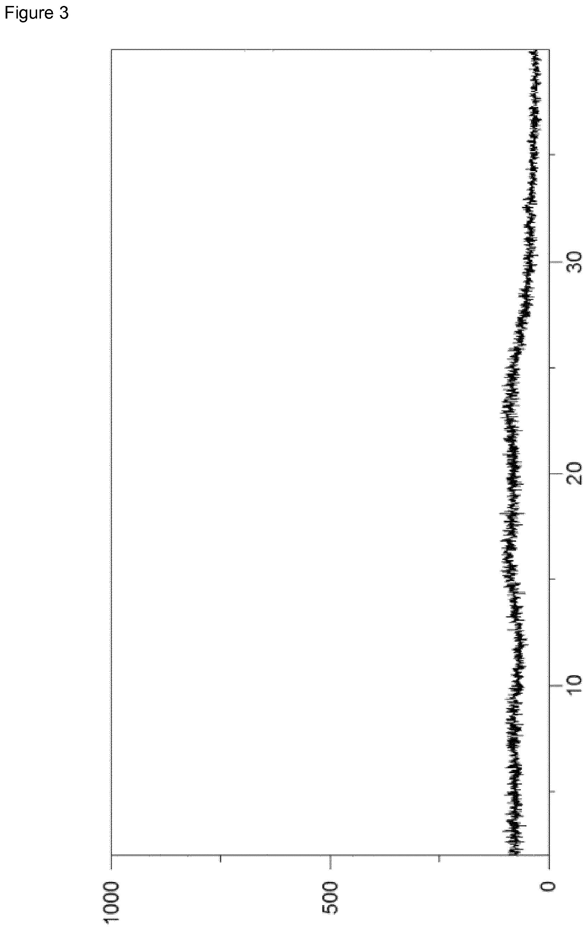

Amorphous elagolix (300 mg, for example prepared according to the procedure disclosed in WO 2009/062087 A1, example 4B) was dissolved in 4.2 mL acetonitrile/water (volume ratio 1:1). Hydrochloric acid fuming (37 w-% aqueous solution, 1.0 mol equivalent, 39.4 microliter) was diluted with 1.5 mL acetonitrile/water (volume ratio 1:1) and added to the elagolix solution. The thus obtained solution was shaken at room temperature and afterwards allowed to stand without shaking for about 10 min before it was filtered with the aid of a syringe filter (pore size 0.45 microns). The homogeneous solution was frozen in a bath of liquid nitrogen and lyophilized at room temperature and a pressure below 2 mbar, yielding acid addition salt of elagolix with hydrochloric acid as a white solid. The obtained material was amorphous as confirmed by the PXRD depicted in Figure 3 herein.

### Reference example 3: (R)-5-(2-fluoro-3-methoxyphenyl)-1-(2-fluoro-6-(trifluoromethyl)benzyl)-6-methyl-3-(2-(2-oxopyrrolidin-1-yl)-2-phenylethyl)pyrimidine-2,4(1H,3H)-dione according Formula (Va)

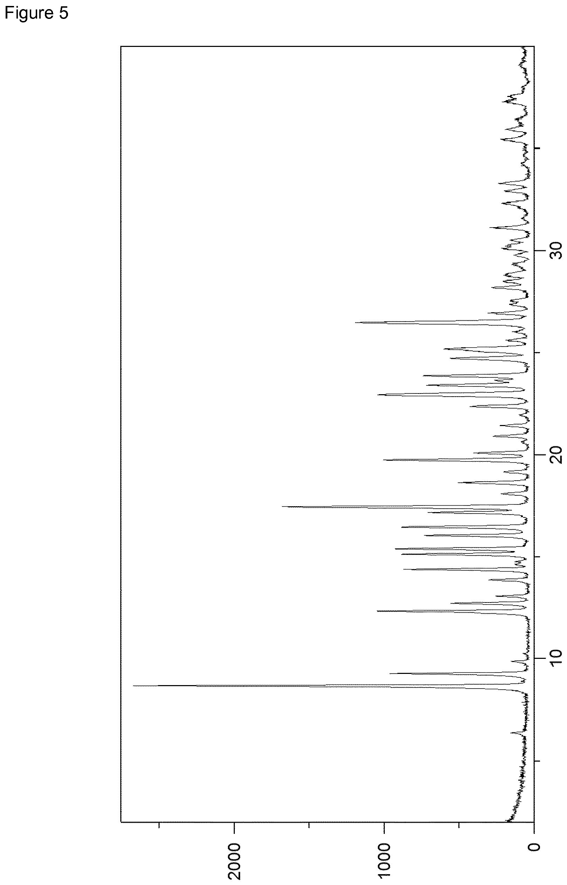

Amorphous elagolix (51 mg, for example prepared according to the procedure disclosed in WO 2009/062087 A1, example 4B) was dissolved in acetone (0.6 mL) at RT. In order to accelerate dissolution the mixture was additionally sonicated in a VWR Ultrasonic Cleaner apparatus. The solution was filtrated with the aid of a syringe filter (pore size 0.45 microns) and stirred at room temperature. After 10 days of stirring precipitation took place yielding to a homogenous suspension. The white solid was collected by filtration and dried at room temperature under vacuum (30 mbar) for 16 hours. The obtained material was crystalline as confirmed by the PXRD depicted in Figure 5 herein.

**Table 2: Reflection list and corresponding relative intensities of crystalline (actually not elagolix but lactam derivative of elagolix) between 2.0 and 30.0° 2-Theta**

| Angle [± 0.2 °2-Theta] | Relative Intensity [%] | Angle [± 0.2 °2-Theta] | Relative Intensity [%] |
|---|---|---|---|
| 6.3 | 4 | 20.6 | 2 |
| 8.7 | 100 | 20.9 | 9 |
| 9.3 | 34 | 21.4 | 7 |
| 9.8 | 4 | 21.9 | 2 |
| 12.3 | 39 | 22.4 | 14 |
| 12.7 | 20 | 22.9 | 38 |
| 13.1 | 8 | 23.4 | 25 |
| 13.8 | 10 | 23.6 | 8 |
| 14.4 | 31 | 23.9 | 26 |
| 14.6 | 3 | 24.7 | 20 |
| 14.7 | 3 | 25.2 | 20 |
| 15.1 | 32 | 25.6 | 4 |
| 15.4 | 34 | 26.0 | 3 |
| 16.0 | 26 | 26.5 | 44 |
| 16.5 | 32 | 26.9 | 10 |
| 17.1 | 25 | 27.3 | 4 |
| 17.4 | 61 | 27.5 | 4 |
| 18.1 | 7 | 28.2 | 9 |
| 18.6 | 16 | 28.5 | 6 |
| 19.1 | 6 | 28.8 | 6 |
| 19.7 | 37 | 29.3 | 3 |
| 20.1 | 13 | 29.7 | 3 |

## Claims

1. Process for the preparation of an acid addition salt of Formula (2) 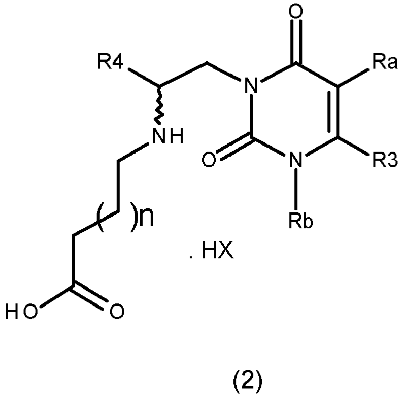 wherein
n = 1 or 2;
Rₐ and R_{b} are independently of each other a substituted or unsubstituted C₁₋₂₀ alkyl, substituted or unsubstituted C₁₋₂₀ alkoxy, substituted or unsubstituted C₁₋₂₀ alkenyl, substituted or unsubstituted C₃₋₁₂ cycloalkyl, substituted or unsubstituted C₆₋₁₂ aryl, substituted or unsubstituted C₅₋₁₂ heteroaryl, substituted or unsubstituted C₇₋₁₂ alkylheteroaryl, substituted or unsubstituted C₇₋₁₂ alkylaryl, substituted or unsubstituted C₃₋₁₂ heterocycloalkyl, substituted or unsubstituted C₅₋₁₂ heterobicycle, substituted or unsubstituted C₅₋₁₂ biaryl, substituted or unsubstituted C₅₋₁₂ bicycloaryl, substituted or unsubstituted C₅₋₁₂ bicycloalkyl, substituted or unsubstituted -C₃₋₇ cycloalkyl-C₃₋₇ heterocycle, substituted or unsubstituted -C₃₋₇ aryl-C₃₋₇ heterocycle, or substituted or unsubstituted -C₃₋₇ heterocycloalkyl-C₃₋₇ heteroaryl;
R₃ = hydrogen or methyl;
R₄ = substituted or unsubstituted phenyl, substituted or unsubstituted C₃₋₇ alkyl, hydrogen, -C₁₋₆ alkanediyl-COOH, or -C(=O)O-(t-butyl);
HX = organic or inorganic acid;
comprising the steps of:
a) dissolving an ester of Formula (3) 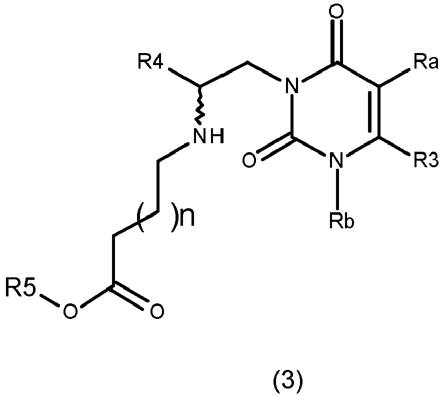 wherein n, Rₐ, R_{b}, R₃, and R₄ are as defined above; and
wherein R₅ is a protective group,
in a solvent;
b) adding an organic or inorganic acid having a pKa of <4.0 to cleave the ester of Formula (3)
c) optionally, adding an anti-solvent;
d) isolating a solid acid addition salt of Formula (2) by filtration; and
e) optionally, drying the solid acid addition salt of Formula (2).

2. The process of claim 1, wherein the acid addition salt has the Formula (II) 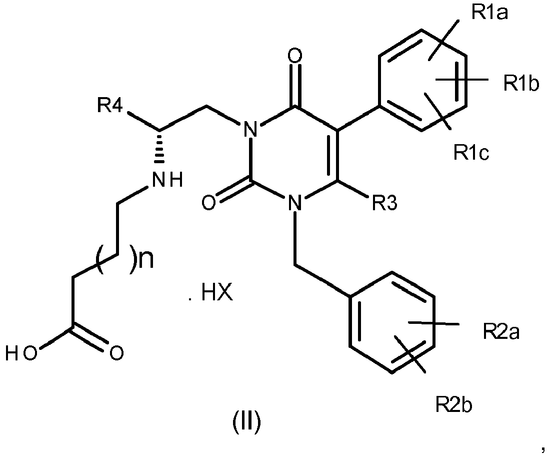 wherein
n = 1 or 2;
R₁ₐ, R_{1b} and R_{1c} are independently selected from hydrogen, halogen, substituted or unsubstituted C₁₋₄ alkyl, hydroxy, substituted or unsubstituted C₁₋₄ alkoxy, or R₁ₐ, and R_{1b} when taken together form -OCH₂O- or -OCH₂CH₂-;
R₂ₐ, and R_{2b} are independently selected from hydrogen, halogen, trifluoromethyl, cyano, or -SO₂CH₃;
R₃ = hydrogen or methyl;
R₄ = substituted or unsubstituted phenyl, substituted or unsubstituted C₃₋₇ alkyl, hydrogen, -C₁₋₆ alkanediyl-COOH, or -C(=O)O-(t-butyl);
HX = organic or inorganic acid, and wherein the process comprises the steps of:
a) dissolving an ester of Formula (III) 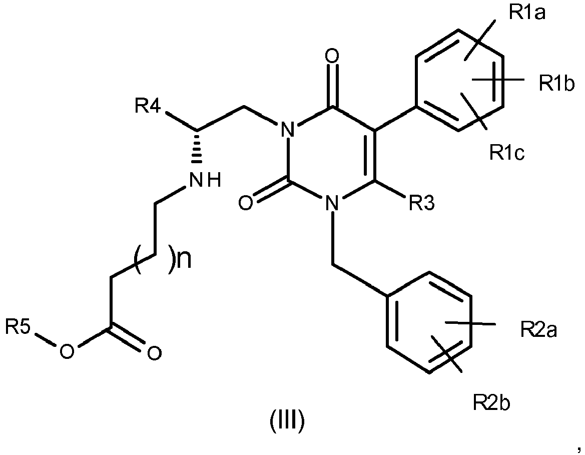 wherein n, R₁ₐ, R_{1b}, R_{1c}, R₂ₐ, R_{2b}, R₃, and R₄ are as defined above; and
wherein R₅ is a protective group,
in a solvent;
b) adding an organic or inorganic acid to cleave the ester of Formula (III)
c) optionally, adding an anti-solvent;
d) isolating a solid acid addition salt of Formula (II) by filtration; and
e) optionally, drying the solid acid addition salt of Formula (II).

3. The process of claim 1 or 2, wherein the protection group R₅ in Formula (3) or Formula (III) is selected from the group consisting of tert-butyl-, triphenylmethyl-, and trialkylsilyl-.

4. The process of any of the preceding claims, wherein the substituents in Formula (II) are defined as follows:
n = 1;
R₁ₐ, = halogen, preferably fluorine;
R_{1b} = unsubstituted C₁₋₄ alkoxy;
R_{1c} = hydrogen;
R₂ₐ, and R_{2b} are independently selected from hydrogen, and halogen;
R₃ = hydrogen or methyl;
R₄ = unsubstituted phenyl or phenyl substituted with halogen, or unsubstituted C₃₋₇ alkyl;
preferably unsubstituted phenyl, or unsubstituted C₃₋₇ alkyl;
HX = organic or inorganic acid, preferably HCl, or H₂SO₄.

5. The process of claim 1 or 3, wherein the acid addition salt of Formula (II) is an acid addition salt of elagolix and has the Formula (IIa) 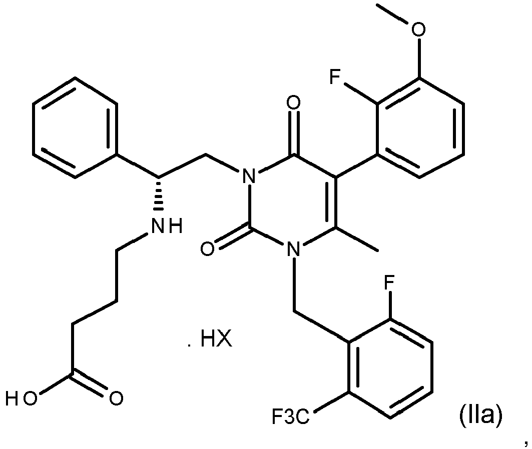 and wherein the ester of Formula (III) is an ester of elagolix and has the Formula (IIIa) 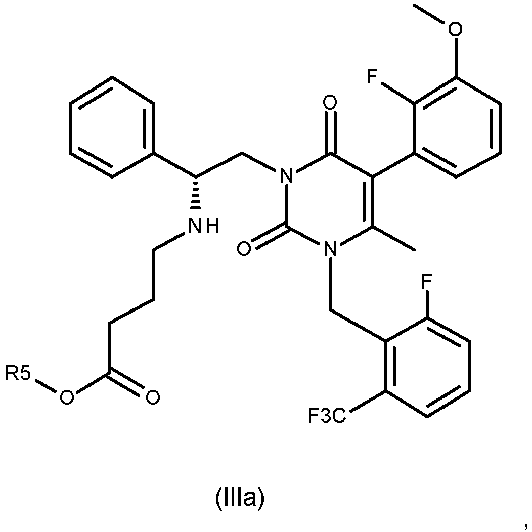 wherein R₅ is as defined in claim 1 or 2.

6. The process of any of the preceding claims, wherein the solvent is isopropanol, ethylacetate (EtOAc) or mixtures thereof.

7. The process of any of the preceding claims, wherein the acid is selected from HCI, trifluoroacetic acid (TFA), HCOOH, H₂SO₄ and mixtures thereof.

8. The process of any of the preceding claims, wherein the ester of Formula (3), preferably Formula (III), further preferred Formula (IIIa), is prepared by a process comprising the steps of:
aa) reacting the primary amine of the compound of Formula (4), preferably Formula (IV), further preferred of Formula (IVa) 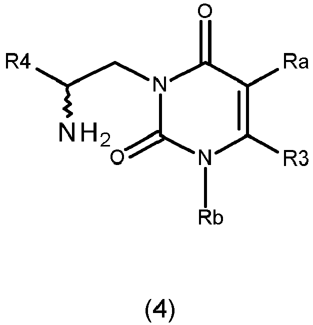 wherein Rₐ, R_{b}, R₃, and R₄ are as defined in claim 1; 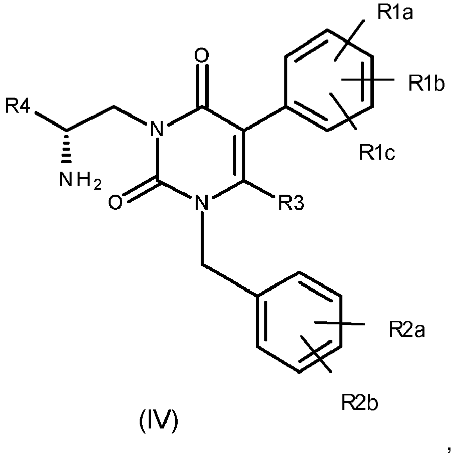 wherein R₁ₐ, R_{1b}, R_{1c}, R₂ₐ, R_{2b}, R₃, and R₄ are as defined in any of claims 2-4; 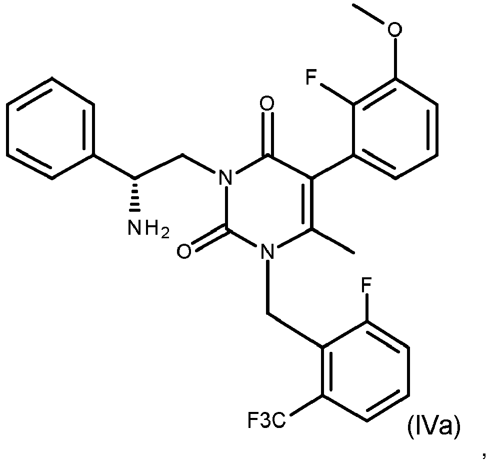 with an ester of 4-halobutyric acid, preferably tert-butyl 4-bromobutyrate, in the presence of a base, preferably K₂CO₃, Na₂CO₃, N-ethyldiisopropylamine (DIEA) or triethylamine (TEA) to provide the compound of Formula (3), preferably Formula (III), further preferred of Formula (IIIa),
bb) optionally, isolating the ester of Formula (3), of Formula (III), preferably of Formula (IIIa).

9. The process of any of one of claims 1 to 7, wherein an acid addition salt of elagolix is isolated, which is an amorphous or crystalline hydrogensulfate salt, or amorphous or crystalline hydrochloride salt.

10. Acid addition salt of Formula (2), Formula (II), or Formula (IIa) as defined in any of the preceding claims, wherein HX is HCl, and H₂SO₄.

11. Acid addition salt of Formula (2), Formula (II), or Formula (IIa) as defined in any of the preceding claims,
wherein the acid addition salt of Formula (2) contains an impurity according to Formula (5) 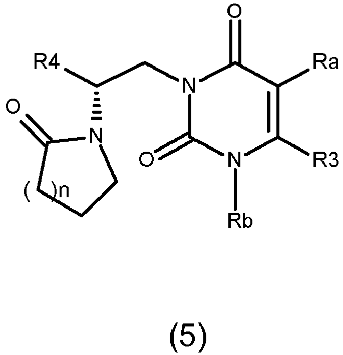 wherein Rₐ, R_{b}, R₃, and R₄ are as defined in claim 1;
wherein the acid addition salt of Formula (II) contains an impurity according to Formula (V) 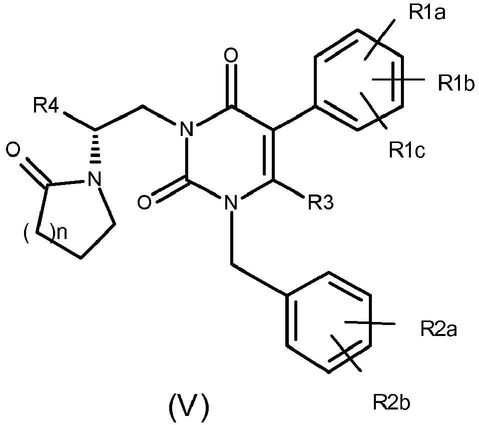 wherein R₁ₐ, R_{1b}, R_{1c}, R₂ₐ, R_{2b}, R₃ and R₄ are as defined for Formula (II) and the acid addition salt of Formula (IIa) contains an impurity according to Formula (Va), respectively, 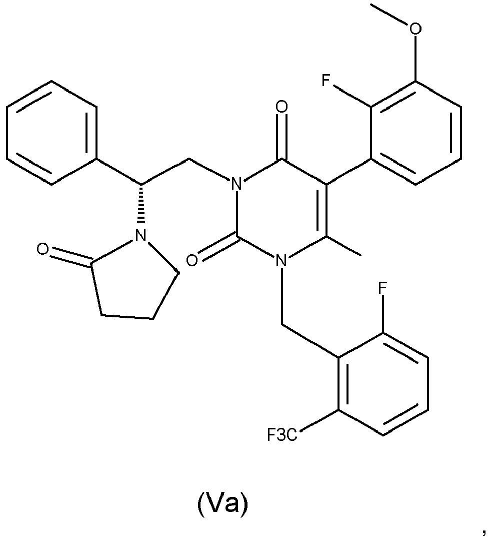 in an amount of less than 1 % by weight.

12. Pharmaceutical composition comprising an acid addition salt of claim 10 or 11 and a pharmaceutically acceptable carrier.

13. Pharmaceutical composition of claim 12 for use as medicament.

14. Pharmaceutical composition of claim 12 for use in a method of treatment or prevention of endometriosis and uterine fibroids.

15. Acid addition salt of Formula (2), Formula (II), or Formula (IIa) of claim 10 or 11 for use as medicament, preferably for use in a method of treatment or prevention of endometriosis and uterine fibroids.
